# EUROPEAN PATENT SPECIFICATION

(11) **EP 2 699 265 B1**
(45) Date of publication and mention of the grant of the patent: **16.10.2019**
(21) Application number: 12715107.4
(22) Date of filing: 19.04.2012
(51) Int. Cl.: A61K 47/12, A61K 47/18, A61K 38/00

(54) **STABLE PHARMACEUTICAL LIQUID FORMULATIONS OF THE FUSION PROTEIN TNFR:Fc**
STABILE PHARMAZEUTISCHE FLÜSSIGFORMULIERUNGEN DES FUSIONSPROTEINS TNFR:FC
FORMULATIONS LIQUIDES PHARMACEUTIQUES STABLES DE LA PROTÉINE DE FUSION TNFR:FC

(30) Priority: 20.04.2011 EP 11163171
(43) Date of publication of application: 26.02.2014
(73) Proprietor: Sandoz AG, 4056 Basel (CH)
(72) Inventor: DEUTEL, Britta, A-6250 Kundl (AT); LAUBER, Thomas, A-6250 Kundl (AT); FUERTINGER, Sabine, A-6250 Kundl (AT)
(74) Representative: Wichmann, Hendrik
(86) International application number: PCT/EP2012/057119
(87) International publication number: WO 2012/143418

(56) References cited:
- WO-A1-2005/012353
- WO-A2-03/072060
- WO-A2-2007/092772
- WO-A2-2011/141926
- SHIRAKI KENTARO ET AL: "Biophysical effect of amino acids on the prevention of protein aggregation", JOURNAL OF BIOCHEMISTRY, JAPANESE BIOCHEMICAL SOCIETY / OUP, TOKYO; JP, vol. 132, no. 4, 1 October 2002 (2002-10-01), pages 591-595, XP002538261, ISSN: 0021-924X

### SEQUENCE LISTING

<110> Sandoz AG
<120> Stable pharmaceutical liquid formulations of the fusion protein TNFR:Fc
<130> EPA-117 965
<160> 4
<170> PatentIn version 3.3
<210> 1
   <211> 455
   <212> PRT
   <213> homo sapiens
<400> 1
<210> 2
   <211> 461
   <212> PRT
   <213> homo sapiens
<400> 2
   Met Ala Pro Val Ala Val Trp Ala Ala Leu Ala Val Gly Leu Glu Leu
   1 5 10 15
   Trp Ala Ala Ala His Ala Leu Pro Ala Gln Val Ala Phe Thr Pro Tyr
   20 25 30
   Ala Pro Glu Pro Gly Ser Thr Cys Arg Leu Arg Glu Tyr Tyr Asp Gln
   35 40 45
   Thr Ala Gln Met Cys Cys Ser Lys Cys Ser Pro Gly Gln His Ala Lys
   50 55 60
   Val Phe Cys Thr Lys Thr Ser Asp Thr Val Cys Asp Ser Cys Glu Asp
   65 70 75 80
   Ser Thr Tyr Thr Gln Leu Trp Asn Trp Val Pro Glu Cys Leu Ser Cys
   85 90 95
   Gly Ser Arg Cys Ser Ser Asp Gln Val Glu Thr Gln Ala Cys Thr Arg
   100 105 110
   Glu Gln Asn Arg Ile Cys Thr Cys Arg Pro Gly Trp Tyr Cys Ala Leu
   115 120 125
   Ser Lys Gln Glu Gly Cys Arg Leu Cys Ala Pro Leu Arg Lys Cys Arg
   130 135 140
   Pro Gly Phe Gly Val Ala Arg Pro Gly Thr Glu Thr Ser Asp Val Val
   145 150 155 160
   Cys Lys Pro Cys Ala Pro Gly Thr Phe Ser Asn Thr Thr Ser Ser Thr
   165 170 175
   Asp Ile Cys Arg Pro His Gln Ile Cys Asn Val Val Ala Ile Pro Gly
   180 185 190
   Asn Ala Ser Met Asp Ala Val Cys Thr Ser Thr Ser Pro Thr Arg Ser
   195 200 205
   Met Ala Pro Gly Ala Val His Leu Pro Gln Pro Val Ser Thr Arg Ser
   210 215 220
   Gln His Thr Gln Pro Thr Pro Glu Pro Ser Thr Ala Pro Ser Thr Ser
   225 230 235 240
   Phe Leu Leu Pro Met Gly Pro Ser Pro Pro Ala Glu Gly Ser Thr Gly
   245 250 255
   Asp Phe Ala Leu Pro Val Gly Leu Ile Val Gly Val Thr Ala Leu Gly
   260 265 270
   Leu Leu Ile Ile Gly Val Val Asn Cys Val Ile Met Thr Gln Val Lys
   275 280 285
   Lys Lys Pro Leu Cys Leu Gln Arg Glu Ala Lys Val Pro His Leu Pro
   290 295 300
   Ala Asp Lys Ala Arg Gly Thr Gln Gly Pro Glu Gln Gln His Leu Leu
   305 310 315 320
   Ile Thr Ala Pro Ser Ser Ser Ser Ser Ser Leu Glu Ser Ser Ala Ser
   325 330 335
   Ala Leu Asp Arg Arg Ala Pro Thr Arg Asn Gln Pro Gln Ala Pro Gly
   340 345 350
   Val Glu Ala Ser Gly Ala Gly Glu Ala Arg Ala Ser Thr Gly Ser Ser
   355 360 365
   Asp Ser Ser Pro Gly Gly His Gly Thr Gln Val Asn Val Thr Cys Ile
   370 375 380
   Val Asn Val Cys Ser Ser Ser Asp His Ser Ser Gln Cys Ser Ser Gln
   385 390 395 400
   Ala Ser Ser Thr Met Gly Asp Thr Asp Ser Ser Pro Ser Glu Ser Pro
   405 410 415
   Lys Asp Glu Gln Val Pro Phe Ser Lys Glu Glu Cys Ala Phe Arg Ser
   420 425 430
   Gln Leu Glu Thr Pro Glu Thr Leu Leu Gly Ser Thr Glu Glu Lys Pro
   435 440 445
   Leu Pro Leu Gly Val Pro Asp Ala Gly Met Lys Pro Ser
   450 455 460
<210> 3
   <211> 330
   <212> PRT
   <213> Homo sapiens
<400> 3
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Glu Glu
   225                 230                 235                 240
   Met Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 4
   <211> 467
   <212> PRT
   <213> artificial
<220>
   <223> Etanercept
<400> 4
   Leu Pro Ala Gln Val Ala Phe Thr Pro Tyr Ala Pro Glu Pro Gly Ser
   1               5                   10                  15
   Thr Cys Arg Leu Arg Glu Tyr Tyr Asp Gln Thr Ala Gln Met Cys Cys
               20                  25                  30
   Ser Lys Cys Ser Pro Gly Gln His Ala Lys Val Phe Cys Thr Lys Thr
           35                  40                  45
   Ser Asp Thr Val Cys Asp Ser Cys Glu Asp Ser Thr Tyr Thr Gln Leu
       50                  55                  60
   Trp Asn Trp Val Pro Glu Cys Leu Ser Cys Gly Ser Arg Cys Ser Ser
   65                  70                  75                  80
   Asp Gln Val Glu Thr Gln Ala Cys Thr Arg Glu Gln Asn Arg Ile Cys
                   85                  90                  95
   Thr Cys Arg Pro Gly Trp Tyr Cys Ala Leu Ser Lys Gln Glu Gly Cys
               100                 105                 110
   Arg Leu Cys Ala Pro Leu Arg Lys Cys Arg Pro Gly Phe Gly Val Ala
           115                 120                 125
   Arg Pro Gly Thr Glu Thr Ser Asp Val Val Cys Lys Pro Cys Ala Pro
       130                 135                 140
   Gly Thr Phe Ser Asn Thr Thr Ser Ser Thr Asp Ile Cys Arg Pro His
   145                 150                 155                 160
   Gln Ile Cys Asn Val Val Ala Ile Pro Gly Asn Ala Ser Met Asp Ala
                   165                 170                 175
   Val Cys Thr Ser Thr Ser Pro Thr Arg Ser Met Ala Pro Gly Ala Val
               180                 185                 190
   His Leu Pro Gln Pro Val Ser Thr Arg Ser Gln His Thr Gln Pro Thr
           195                 200                 205
   Pro Glu Pro Ser Thr Ala Pro Ser Thr Ser Phe Leu Leu Pro Met Gly
       210                 215                 220
   Pro Ser Pro Pro Ala Glu Gly Ser Thr Gly Asp Glu Pro Lys Ser Cys
   225                 230                 235                 240
   Asp Lys Thr His Thr Cys Pro Pro Cys Pro Ala Pro Glu Leu Leu Gly
                   245                 250                 255
   Gly Pro Ser Val Phe Leu Phe Pro Pro Lys Pro Lys Asp Thr Leu Met
               260                 265                 270
   Ile Ser Arg Thr Pro Glu Val Thr Cys Val Val Val Asp Val Ser His
           275                 280                 285
   Glu Asp Pro Glu Val Lys Phe Asn Trp Tyr Val Asp Gly Val Glu Val
       290                 295                 300
   His Asn Ala Lys Thr Lys Pro Arg Glu Glu Gln Tyr Asn Ser Thr Tyr
   305                 310                 315                 320
   Arg Val Val Ser Val Leu Thr Val Leu His Gln Asp Trp Leu Asn Gly
                           325                 330                 335
   Lys Glu Tyr Lys Cys Lys Val Ser Asn Lys Ala Leu Pro Ala Pro Ile
               340                 345                 350
   Glu Lys Thr Ile Ser Lys Ala Lys Gly Gln Pro Arg Glu Pro Gln Val
           355                 360                 365
   Tyr Thr Leu Pro Pro Ser Arg Glu Glu Met Thr Lys Asn Gln Val Ser
       370                 375                 380
   Leu Thr Cys Leu Val Lys Gly Phe Tyr Pro Ser Asp Ile Ala Val Glu
   385                 390                 395                 400
   Trp Glu Ser Asn Gly Gln Pro Glu Asn Asn Tyr Lys Thr Thr Pro Pro
                   405                 410                 415
   Val Leu Asp Ser Asp Gly Ser Phe Phe Leu Tyr Ser Lys Leu Thr Val
               420                 425                 430
   Asp Lys Ser Arg Trp Gln Gln Gly Asn Val Phe Ser Cys Ser Val Met
           435                 440                 445
   His Glu Ala Leu His Asn His Tyr Thr Gln Lys Ser Leu Ser Leu Ser
       450                 455                 460
   Pro Gly Lys
   465

## Description

The present invention relates to stable pharmaceutical liquid formulations of the fusion protein TNFR:Fc comprising different buffer systems and stabilizers as defined in the claims. In particular, it could be demonstrated that the physical stability of TNFR:Fc is significantly improved by using a citrate buffer system and lysine and/or proline as stabilizer, as defined in the claims.

### BACKGROUND OF THE INVENTION

Commercial antibodies are commonly formulated in phosphate buffer. Also TNFR:Fc is commonly buffered in sodium phosphate (EP1478394, WO 03/072060 A2). Currently, e.g. the TNFR:Fc protein Etanercept is marketed under the tradename Enbrel® having a composition as shown in Table 1.

**Table 1 Composition of Etanercept (Enbrel®)**

| | [mg/mL] | [mM] |
|---|---|---|
| Etanercept | 50 | 0,3 |
| Sucrose | 10 | 29 |
| Sodium chloride | 5.8 | 100 |
| L-arginine hydrochloride | 5.3 | 25 |
| Sodium phosphate monobasic | 2.6 | 19 |
| Sodium phosphate dibasic | 0.9 | 6 |
| WFI | ad 0.5 /1.0 ml | |
| pH | 6.3 ± 0.2 | |

Aggregation of antibody products can be controlled by the addition of small amphiphilic molecules. Thereof, L-arginine is the amino acid of choice in suppressing protein interactions in commercial formulations (Baynes et al (2005) 44(12):4919-25; EP1478394). Being a polar additive, it prevents the aggregation of protein folding intermediates.

Like L-arginine, L-lysine is capable of significantly preventing heat- and dilution-induced aggregration of lysozyme (Shiraki et al (2002) J Biochem, 132(4):591-5).

L-proline has been established as a stabilizer in liquid immunoglobulin preparations like Sandoglobulin® or Privigen®. As an hydrophobic amino acid, it is assumed to

interfere with hydrophobic protein-protein interactions and thus protects IgG from denaturation and aggregation. Besides, L-proline exhibits a good safety record when administered to patients with primary immunodeficiencies and was found to represent an amino acid of low toxicity in animal studies (Bolli et al, (2001) Biologicals, 38(1):150-7).

Recent studies contemplate citrate buffer as beneficial in monoclonal antibody formulations as it efficiently minimizes degradations like asparagine deamidations (Zheng and Janis, (2006) Int J Pharm, 308(1-2):46-51). Another advantage of citrate buffer is its capacity to stabilize pH during freezing while the established phosphate buffer system shows the greatest change in pH when lowering temperatures from +25 to -30 degrees C (Kolhe et al, (2010) Biotechnol Prog, 26(3):727-33).

WO 2007/092772 A2 discloses compositions for proteins comprising a variant, non-naturally occurring Fc regions. WO 2005/012353 A1 discloses a method for producing etanercept protein crystals.

Fusion proteins may generate a variety of degraded and aggregated products which subsequently may lead to reduced activity and even adverse effects like immunogenicity. Thus, there is still a need for a stable liquid formulation of the fusion protein TNFR:Fc.

Such a formulation shall fulfil a variety of tasks. It has to be physiologically acceptable and preferably provides an environment which guarantees stability of the biopharmaceutical drug in a therapeutically effective concentration. Furthermore, the formulation shall enable a satisfactory shelf-life of the drug.

It is thus the object of the present invention to provide pharmaceutical formulations for TNFR:Fc which can be used as an alternative to those formulations known from prior art. Another object of the present invention is to provide pharmaceutical formulations for TNFR:Fc which are advantageous compared to formulations known from prior art. It is yet another object of the present invention to provide pharmaceutical formulations for TNFR:Fc which cause less drug aggregation than formulations known from prior art.

The present invention demonstrates that, by replacing, e.g., the commonly used phosphate buffer to a citrate buffer system and the stabilizer arginine to lysine, the physical stability of TNFR:Fc can be significantly improved. The proposed buffer citric acid and stabilizer lysine protect TNFR:Fc against degradation induced by mechanical and temperature stress (25 and 40°C) and at intended storage at 2-8°C protein degradation was significantly lower in proposed formulations compared to the commonly used phosphate buffered formulations. Therefore, the quality parameters relating to physical stability of the product could be improved. The increased physical stability of the drug product enables a prolonged shelf-life compared to the common product formulations and ensures product safety.

### SUMMARY OF THE INVENTION

In a first aspect, the invention relates to a pharmaceutical composition, comprising 0.15 mM to 0.5 mM TNFR:Fc, a citrate buffer at a concentration from 25 mM to 120 mM at a pH from 6.2 to 6.4, and an amino acid at a concentration from 15 mM to 100 mM selected from the group consisting of lysine and proline and their pharmaceutical acceptable salts; wherein TNFR:Fc is etanercept.

In a second aspect, the invention pertains to a kit comprising a composition according to the first aspect and instructions for use of said composition.

In still a third aspect, the invention relates to a method of producing a pharmaceutical composition according to the first aspect, comprising combining TNFR:Fc, a citrate buffer and an amino acid selected from the group consisting of lysine and proline and their pharmaceutical acceptable salts.

In a final aspect, the invention also relates to a composition according to the first aspect for use in a medical treatment, in particular in a treatment of a disease selected from an autoimmune disease, ankylosing spondylitis, juvenile rheumatoid arthritis, psoriasis, psoriatic arthritis, rheumatoid arthritis, Wegener's disease (granulomatosis), Crohn's disease (or inflammatory bowel disease), chronic obstructive pulmonary disease (COPD), Hepatitis C, endometriosis, asthma, cachexia, atopic dermatitis, Alzheimer and cancer.

### DETAILED DESCRIPTION OF THE PREFERRED EMBODIMENTS

The formation of degradation products during storage of TNFR:Fc seems to be the most critical attribute of the molecule. TNFR:Fc in citrate formulations displays in general a lower degradation potential, which could be due to the greater net charge of sodium citrate compared to sodium phosphate and therefore possible interaction with the charged TNFR:Fc molecule.

Accordingly, in a first aspect, the invention relates to a pharmaceutical composition, comprising 0.15 mM to 0.5 mM TNFR:Fc, a citrate buffer at a concentration from 25 mM to 120 mM at a pH from 6.2 to 6.4, and an amino acid at a concentration from 15 mM to 100 mM selected from the group consisting of lysine and proline and their pharmaceutical acceptable salts; wherein TNFR:Fc is etanercept.

Tumor Necrosis Factor alpha (TNF-alpha) is a member of a group of cytokines that stimulate the acute phase reaction, and thus is a cytokine involved in systemic inflammation. TNF-alpha is able to induce inflammation, induce apoptotic cell death, and to inhibit tumorgenesis and viral replication. Dysregulation of TNF-alpha production has been implicated in a variety of human diseases like autoimmune disease, ankylosing spondylitis, juvenile rheumatoid arthritis, psoriasis, psoriatic arthritis, rheumatoid arthritis, Wegener's disease (granulomatosis), Crohn's disease or inflammatory bowel disease, chronic obstructive pulmonary disease (COPD), Hepatitis C, endometriosis, asthma, cachexia, atopic dermatitis, Alzheimer as well as cancer.

Its receptor molecules include, TNFR1 (TNF receptor type 1; CD120a; p55/60; for human: RefSeq (mRNA): NM_001065, RefSeq (protein): NP_001056 (SEQ ID NO:1)) and TNFR2 (TNF receptor type 2; CD120b; p75/80; for human: RefSeq (mRNA): NM_001066, RefSeq (protein): NP_001057 (SEQ ID NO:2)). TNF-R1 is expressed in most tissues and can be fully activated by both the membrane-bound and soluble trimeric forms of TNF, whereas TNF-R2 is found only in cells of the immune system and responds to the membrane-bound form of the TNF homotrimer. Upon contact with TNF-alpha, TNF receptors form trimers and thereby initiate intracellular cell signaling.

Accordingly, soluble TNFR molecules or fragments thereof, which are able to bind to TNF-alpha, can be used as a competitive inhibitor for TNF-alpha. The present invention relates to such soluble TNFR molecules fused to an Fc portion of a human immunoglobulin (TNFR:Fc).

In the context of the present disclosure, the TNFR part of TNFR:Fc refers to any TNFR polypeptide having at least 90%, preferably at least 91 %, such as at least 92 % or at least 93 %, more preferably at least 94 %, such as at least 95 %, or at least 96 %, even more preferably at least 97 %, such as at least 98 %, or at least 99 %, and most preferably 100 % identity to an amino acid sequence comprising at least 150-250, preferably at least 175-245 of TNFR1 or TNFR2, preferably TNFR2, more preferably 200-240, and most preferably 225-235 amino acids of the extracellular part of TNFR2, and still binding to TNF-alpha, as determined by ELISA or any other convenient assay. More preferably, the said TNFR is capable of binding to TNF-alpha and Lymphotoxin alpha (LT-alpha), as determined by ELISA or any other convenient assay. Such assays are well-known to the skilled person.

Generally, a polypeptide has "at least x % identity" over a defined length of amino acids with another polypeptide if the sequence in question is aligned with the best matching sequence of the amino acid sequence and the sequence identity between those to aligned sequences is at least x %. Such an alignment can be performed using for example publicly available computer homology programs such as the "BLAST" program, such as "blastp" provided at the NCBI homepage at http://www.ncbi.nlm.nih.gov/blast/blast.cgi, using the default settings provided therein. Further methods of calculating sequence identity percentages of sets of polypeptides are known in the art.

The Fc-region (fragment crystallisable region) refers to the tail region of an antibody, in the case of IgG composed of the second and third constant domain of the antibody's two heavy chains. In certain embodiments, the Fc polypeptide comprises the constant region of an IgG class heavy chain or a fragment and/or variant thereof and in other embodiments the constant region of other immunoglobulin isotypes can be used to generate such TNFR:Fc fusions. For example, a TNFR:Fc polypeptide comprising the constant region of an IgM class heavy chain or a fragment and/or variant thereof could be used. Preferably, the Fc fragment is derived from IgG, more preferably from IgG1, even more preferably from human IgG1. The constant region of immunoglobulin heavy chains, with a specific example of a human IgG1 class heavy chain constant domain provided by SEQ ID NO: 3, comprises a CH1 domain (amino acids 1 through 98 of SEQ ID NO:3), a hinge region (amino acids 99 through 110 of SEQ ID NO:3), a CH2 domain (amino acids 111 through 223 of SEQ ID NO:3), and a CH3 domain (amino acids 224 through 330 of SEQ ID NO:3). As used herein, an Fc domain can contain one or all of the heavy chain CH1, hinge, CH2, and CH3 domains described above, or fragments or variants thereof. Certain embodiments of the invention include TNFR:Fc comprising all or a portion of the extracellular domain of TNFR1 (SEQ ID NO: 1) or TNFR2 (SEQ ID NO:2) fused to all or a portion of SEQ ID NO:3, optionally with a linker polypeptide between the TNFR portion and the Fc portion of the TNFR:Fc. For example, CH1, CH2 and the entire hinge region may be present in the molecule. In furtherincidents, a heavy chain constant region comprising at least a portion of CH1 is the Fc portion of a TNFR:Fc. Certain incidents can also include, for example, all of the hinge region or the C-terminal half of the hinge region to provide a disulfide bridge between heavy chains. For example, CH1 may be present along with the first seven amino acids of the hinge (amino acids 99 through 105 of SEQ ID NO: 3). In certainincidents, the TNFR polypeptide is covalently linked, optionally through a polypeptide linker, to the N-terminus of at least one portion of a CH1 region of a heavy chain constant domain to form a TNFR:Fc.

If a dimeric TNFR:Fc is desired, it is important to include the portion of the hinge region implicated in disulfide bond formation between the heavy chains (for example, a portion of amino acids 99 through 110 of SEQ ID NO: 3 that includes amino acid 109 of SEQ ID NO: 3). In further incidents, the TNFR:Fc can comprise portions of the CH3 domain that do not include the C-terminal lysine residue (amino acid 330 of SEQ ID NO: 3), as this residue has been observed to be removed in post-translational processing of IgG heavy chain polypeptides. Fc fusions and Fc fragments are well-known in the art.

In the present invention, the TNFR:Fc is Etanercept.

Etanercept is a dimer of two molecules of the extracellular portion of the p75 TNF-alpha receptor, each molecule consisting of a 235 amino acid TNFR-derived polypeptide that is fused to a 232 amino acid Fc portion of human IgG1. The amino acid sequence of the monomeric component of etanercept is shown as SEQ ID NO:4. In the dimeric form of this molecule, two of these fusion polypeptides (or "monomers") are held together by three disulfide bonds that form between the immunoglobulin portions of the two monomers. The etanercept dimer therefore consists of 934 amino acids, and has an apparent molecular weight of approximately 150 kilodaltons. In North America, etanercept is co-marketed by Amgen and Pfizer under the trade name Enbrel® in two separate formulations, one in powder form, the other as a pre-mixed liquid. Wyeth is the sole marketer of Enbrel® outside of North America excluding Japan where Takeda Pharmaceuticals markets the drug.

The TNFR:Fc may be recombinantly produced, preferably by using a mammalian cell based expression system. Preferably, said mammalian cell-based expression system is at least one selected from the group consisting of Baby hamster Kidney cell lines (e.g., BHK21); Chinese hamster ovary cell lines (e.g., CHO-K1, CHO-DG44, CHO-DXB, or CHO-dhfr-); Murine myeloma cell lines (e.g., SP2/0); Mouse myeloma cell lines (e.g., NS0); Human embryonic kidney cell lines (e.g., HEK-293); Human-retina-derived cell lines (e.g., PER-C6), and/or Amniocyte cell lines (e.g., CAP). Preferably, hamster cell based expression systems are being used. BHK21 ("Baby Hamster Kidney") cells belong to a quasi diploid established line of Syrian hamster cells, descended from a clone from an unusually rapidly growing primary culture of newborn hamster kidney tissue. Non limiting examples for BHK-21 cell lines which are commercially available and can be used in the context of the present invention are BHK-21 (C-13); BHK21-pcDNA3.1-HC; BHK570; Flp-In-BHK Cell Line; and/or BHK 21 (Clone 13) hamster cell line.

Chinese hamster ovary (CHO) cells are a cell line derived from the ovary of the Chinese hamster. They are often used in biological and medical research and are commercially utilized in the production of therapeutic proteins. They were introduced in the 1960s and were originally grown as a monolayer culture. Today, CHO cells are the most commonly used mammalian hosts for industrial production of recombinant protein therapeutics and are usually grown in suspension culture.

Non limiting examples for CHO cell lines which are commercially available and can be used in the context of the present invention are FreeStyle CHO-S cells; ER-CHO Cell Line; CHO 1-15 500 CHINESE HAM; CHO-DXB, CHO-dhfr-, CHO DP-12 clone#1934; CHO-CD36; CHO-ICAM-1; CHO-K1; Ovary; HuZP3-CHOLec3.2.8.1; xrs5; CHO-K1/BB2 Cells; CHO-K1/BB3 Cells; CHO-K1/EDG8/Galpha15 Cells; CHO-K1/M5 Cells; CHO-K1/NK1 Cells; CHO-K1/NK3 Cells; CHO-K1/NMUR1 Cells; CHO-K1/NTSR1 Cells; CHO-K1/OX1 Cells; CHO-K1/PAC1/Gα15 Cells; CHO-K1/PTAFR Cells; CHO-K1/TRH1 Cells; CHO-K1/V1B Cells; 5HT1A Galpha-15-NFAT-BLA CHO-K1 Cell Line; AVPR2 CRE-BLA CHO-K1 Cell Line; CHO-S Cells SFM Adapted; DG44 Cells; Flp-In-CHO Cell Line; GeneSwitch-CHO Cell Line; NFAT-bla CHO-K1 Cell Line; T-REx-CHO Cell Line; GenoStat CHO K-1 Stable Cell Line; GenoStat CHO K-1 Stable Cell Line Kit; CHO-K1 Cell Line hamster, CHO-PEPT1 Cell line, CHO SSF3 and/or CHO-HPT1 Cell Line. In a particularly preferred embodiment, the hamster cell-based expression system is a CHO-dhfr- cell line.

The pharmaceutical composition comprises TNFR:Fc at a concentration from 0.15 mM to 0.5 mM, such as 0.4 mM or 0.45 mM, more preferably at a concentration from 0.25 mM to 0.35 mM, such as about 0.3 mM.

The citrate buffer may be any suitable citrate buffer. For example, the citrate buffer may comprise or consist of sodium citrate, potassium citrate, citric acid, or mixtures thereof. The citrate buffer seems to have the greatest influence on the stability of the formulation. The increased stability of a 50 mM citrate buffer formulation compared to 25 mM citrate buffer formulation is shown in the examples. An increase up to at least 120 mM could lead to even increased effects. A minimum of 25 mM citrate buffer seems to be mandatory for the stabilization. Accordingly, the pharmaceutical composition comprises the citrate buffer at a concentration from 25 mM to 120 mM, such as from 30 mM to 115 mM, preferably at a concentration from 40 mM to 110 mM, such as from 45 mM to 105 mM, more preferably at a concentration from 50 mM to 100 mM, such as at a concentration of 55 mM, 60 mM, 65 mM, 70 mM, 75 mM, 80 mM, 85 mM, 90 mM, or 95 mM. In another preferred embodiment, the pharmaceutical composition comprises the citrate buffer at a concentration as indicated in the compositions described in the Examples section.

The pH is from 6.2 to 6.4, such as about 6.3.

The pharmaceutical composition comprises an amino acid selected from the group consisting of lysine and proline and their pharmaceutical acceptable salts, such as hydrochlorides. The amino acid may be in D-, L- or DL-configuration, preferably in L-configuration.

At the addition of about 25 mM the additional effect of the combination with the basic amino acid lysine is striking. The addition of up to 100 mM lysine is believed to have an additional effect. Thus, the pharmaceutical composition comprises the amino acid at a concentration from 15 mM to 100 mM, preferably at a concentration from 20 mM to 90 mM, more preferably at a concentration from 25 mM to 75 mM. In another preferred embodiment, the pharmaceutical composition comprises the amino acid at a concentration as indicated in the compositions described in the Examples section. In a particularly preferred embodiment, the amino acid is lysine, or its pharmaceutical acceptable salts.

In addition, the pharmaceutical composition may further comprise at least one tonicity modifier. As used herein, the term "tonicity modifier" is intended to describe a molecule other than citrate, lysine or proline that contributes to the osmolality of a solution. Preferably, the osmolality of a pharmaceutical composition is regulated in order to stabilize the active ingredient and in order to minimize the discomfort to the patient upon administration. Generally, it is preferred that a pharmaceutical composition be isotonic with serum by having the same or similar osmolality, i.e. an osmolality from about 180 to 480 mosmol/kg. Preferably, the at least one tonicity modifier is selected from the group consisting of sodium chloride, cysteine, histidine, glycine, potassium chloride, sucrose, glucose and mannitol, more preferably the tonicity modifier is sodium chloride and/or sucrose. The pharmaceutical composition may comprise the at least one tonicity modifier at a total concentration from 5 mM to 200 mM, such as from 10 mM to 190 mM, from 15 mM to 180 mM, from 20 mM to 170 mM, from 25 mM to 160 mM, from 30 mM to 150 mM, from 35 mM to 140 mM, from 40 mM to 130 mM, or from 45 mM to 120 mM, e.g. 110 mM, but preferably at a concentration from 50 mM to 100 mM. In another preferred embodiment, the pharmaceutical composition comprises the tonicity modifier(s) at a concentration as indicated in the compositions described in the Examples section.

Further, the pharmaceutical composition may comprise at least one excipient. The term "excipient" as used herein refers to a pharmacologically inactive substance used as a carrier for the active agent in a pharmaceutical composition. In some cases, an "active" substance may not be easily administered and absorbed by the human body. In such cases the substance in question may be mixed with an excipient or dissolved in an excipient solution. Excipients are also sometimes used to bulk up formulations that contain very potent active ingredients, in order to facilitate a convenient and accurate dosing. In addition to their use in the single-dosage quantity, excipients can be used in the manufacturing process to optimize the handling of the concerned active substance. Depending on the route of administration and the form of the pharmaceutical composition, different excipients may be used. Thus, excipients may comprise inter alia antiadherents, binders, colours and preservatives such as antioxidants.

For example, at least one excipient may be selected from the group consisting of lactose, glycerol, xylitol, sorbitol, mannitol, maltose, inositol, trehalose, glucose, bovine serum albumin (BSA), dextran, polyvinyl acetate (PVA), hydroxypropyl methylcellulose (HPMC), polyethyleneimine (PEI), gelatin, polyvinylpyrrolidone (PVP), hydroxyethylcellulose (HEC), polyethylene glycol (PEG), ethylene glycol, glycerol, dimethysulfoxide (DMSO), dimethylformamide (DMF), L-serine, sodium glutamate, alanine, glycine, sarcosine, gamma-aminobutyric acid (GABA), polyoxyethylene sorbitan monolaurate (Tween-20), polyoxyethylene sorbitan monooleate (Tween-80), sodium dodecyl sulphate (SDS), polysorbate, polyoxyethylene copolymer, potassium phosphate, sodium acetate, ammonium sulfate, magnesium sulfate, sodium sulfate, trimethylamine N-oxide, betaine, zinc ions, copper ions, calcium ions, manganese ions, magnesium ions, CHAPS, sucrose monolaurate and 2-O-beta-mannoglycerate. In one preferred embodiment, the excipient may be chosen from those described in the Examples section.

The pharmaceutical composition may comprise the at least one excipient at a total concentration of at least 0.1 mM, e.g. from 0.1 mM to 0.7 mM, such as at a concentration of 0.6 mM, preferably at a concentration from 0.15 mM to 0.5 mM, such as from 0.2 mM to 0.4 mM, more preferably at a concentration from 0.24 mM to 0.34 mM. In another preferred embodiment, the pharmaceutical composition comprises the excipient at a concentration as indicated in the compositions described in the Examples section.

Preferably, the composition is liquid. However, in another embodiment, the pharmaceutical composition may be lyophilized, and can be reconstituted, for example by the addition of water, forming a liquid composition. Hence, the pharmaceutical composition may further comprise a pharmaceutically acceptable solvent. In a preferred embodiment, the pharmaceutically acceptable solvent is water. The concentrations of components presented herein refer to a liquid formulation as well as to a constituted lyophilate or a formulation to be lyophilised.

Excipients might display a protective effect during freezing of lyophilized formulations, so called cryoprotective features. Furthermore, metal chelating agents and tensides can be added. Some agents may have a double role, e.g., some sugars or sugar alcohols can serve for example as excipient, cryoprotective and/or tonifying agent. The present formulation in aqueous state is ready to use, while the lyophilized state of the the present formulation can be transferred into liquid formulations by e.g. addition of water for injection.

Particularly preferred compositions comprise or consist of 0.15 mM to 0.5 mM Etanercept, 25 mM to 120 mM citrate buffer, e.g. sodium citrate, 15 mM to 100 mM lysine, e.g. lysine hydrochloride, 10 mM to 100 mM sucrose and 5 mM to 200 mM sodium chloride at a pH value of about 6.3.

Alternatively, the pharmaceutical composition may comprise or consist of 0.3 mM Etanercept, 50 mM citrate buffer, e.g. sodium citrate, 25 mM lysine, e.g. lysine hydrochloride, 29 mM sucrose and 75 mM sodium chloride at a pH value of about 6.3.

In still another preferred embodiment, the pharmaceutical composition may comprise or consist of 0.3 mM Etanercept, 25 mM citrate buffer, e.g. sodium citrate, 25 mM lysine, e.g. lysine hydrochloride, 29 mM sucrose and 88 mM sodium chloride at a pH value of about 6.3.

However, the pharmaceutical composition may also comprise or consist of 0.15 mM to 0.5 mM Etanercept, 25 mM to 120 mM citrate buffer, e.g. sodium citrate, 15 mM to 100 mM proline, 10 mM to 100 mM sucrose and 5 mM to 200 mM sodium chloride at a pH value of about 6.3.

Finally, the pharmaceutical composition may also comprise or consist of 0.3 mM Etanercept, 25 mM citrate buffer, e.g. sodium citrate, 25 mM proline, 29 mM sucrose and 75 mM sodium chloride at a pH value of about 6.3.

The pharmaceutical composition may also comprise or consist of 0.3 mM Etanercept, 50 mM citrate buffer, e.g. sodium citrate, 50 mM lysine, e.g. lysine hydrochloride, 29 mM sucrose and 75 mM sodium chloride at a pH value of about 6.3.

The pharmaceutical composition may also comprise or consist of 0.3 mM Etanercept, 120 mM citrate buffer, e.g. sodium citrate, 25 mM lysine, e.g. lysine hydrochloride, 29 mM sucrose and 75 mM sodium chloride at a pH value of about 6.3.

The pharmaceutical composition may also comprise or consist of 0.3 mM Etanercept, 120 mM citrate buffer, e.g. sodium citrate, 50 mM lysine, e.g. lysine hydrochloride, 29 mM sucrose and 75 mM sodium chloride at a pH value of about 6.3.

The pharmaceutical composition may also comprise or consist of 0.3 mM Etanercept, 50 mM citrate buffer, e.g. sodium citrate, 50 mM lysine, e.g. lysine hydrochloride, 29 mM sucrose and 51 mM sodium chloride at a pH value of about 6.3.

The pharmaceutical composition may also comprise or consist of 0.3 mM Etanercept, 120 mM citrate buffer, e.g. sodium citrate, 25 mM lysine, e.g. lysine hydrochloride, 29 mM sucrose and 22 mM sodium chloride at a pH value of about 6.3.

The pharmaceutical composition may also comprise or consist of 0.3 mM Etanercept, 120 mM citrate buffer, e.g. sodium citrate, 50 mM lysine, e.g. lysine hydrochloride and 29 mM sucrose at a pH value of about 6.3.

The pharmaceutical composition may also comprise or consist of 0.3 mM Etanercept, 50 mM citrate buffer, e.g. sodium citrate, 50 mM lysine, e.g. lysine hydrochloride and 75 mM sodium chloride at a pH value of about 6.3.

The pharmaceutical composition may also comprise or consist of 0.3 mM Etanercept, 120 mM citrate buffer, e.g. sodium citrate, 25 mM lysine, e.g. lysine hydrochloride and 36 mM sodium chloride at a pH value of about 6.3.

The pharmaceutical composition may also comprise or consist of 0.3 mM Etanercept, 120 mM citrate buffer, e.g. sodium citrate, 50 mM lysine, e.g. lysine hydrochloride and 17 mM sodium chloride at a pH value of about 6.3.

The pharmaceutical composition may also comprise or consist of 0.3 mM Etanercept, 50 mM citrate buffer, e.g. sodium citrate, 50 mM lysine, e.g. lysine hydrochloride, 29 mM sucrose and 75 mM sodium chloride at a pH value of about 6.3.

The pharmaceutical composition may also comprise or consist of 0.3 mM Etanercept, 75 mM citrate buffer, e.g. sodium citrate, 25 mM lysine, e.g. lysine hydrochloride, 29 mM sucrose and 75 mM sodium chloride at a pH value of about 6.3.

The pharmaceutical composition may also comprise or consist of 0.3 mM Etanercept, 75 mM citrate buffer, e.g. sodium citrate, 50 mM lysine, e.g. lysine hydrochloride, 29 mM sucrose and 75 mM sodium chloride at a pH value of about 6.3.

The pharmaceutical composition may also comprise or consist of 0.3 mM Etanercept, 75 mM citrate buffer, e.g. sodium citrate, 25 mM lysine, e.g. lysine hydrochloride, 29 mM sucrose and 56 mM sodium chloride at a pH value of about 6.3.

The pharmaceutical composition may also comprise or consist of 0.3 mM Etanercept, 75 mM citrate buffer, e.g. sodium citrate, 50 mM lysine, e.g. lysine hydrochloride, 29 mM sucrose and 31 mM sodium chloride at a pH value of about 6.3.

The pharmaceutical composition may also comprise or consist of 0.3 mM Etanercept, 75 mM citrate buffer, e.g. sodium citrate, 25 mM lysine, e.g. lysine hydrochloride, 19 mM sucrose and 75 mM sodium chloride at a pH value of about 6.3.

The pharmaceutical composition may also comprise or consist of 0.3 mM Etanercept, 75 mM citrate buffer, e.g. sodium citrate, 50 mM lysine, e.g. lysine hydrochloride and 59 mM sodium chloride at a pH value of about 6.3.

The pharmaceutical composition may also comprise or consist of 0.3 mM Etanercept, 25 mM citrate buffer, e.g. sodium citrate, 25 mM lysine, e.g. lysine hydrochloride, 29 mM sucrose and 75 mM sodium chloride at a pH value of about 6.3.

The pharmaceutical composition may also comprise or consist of 0.3 mM Etanercept, 25 mM citrate buffer, e.g. sodium citrate, 50 mM lysine, e.g. lysine hydrochloride, 29 mM sucrose and 75 mM sodium chloride at a pH value of about 6.3.

The pharmaceutical composition may also comprise or consist of 0.3 mM Etanercept, 50 mM citrate buffer, e.g. sodium citrate, 25 mM lysine, e.g. lysine hydrochloride, 29 mM sucrose and 75 mM sodium chloride at a pH value of about 6.3.

The pharmaceutical composition may also comprise or consist of 0.3 mM Etanercept, 50 mM citrate buffer, e.g. sodium citrate, 50 mM lysine, e.g. lysine hydrochloride, 29 mM sucrose and 75 mM sodium chloride at a pH value of about 6.3.

The pharmaceutical composition may also comprise or consist of 0.3 mM Etanercept, 50 mM citrate buffer, e.g. sodium citrate, 50 mM lysine, e.g. lysine hydrochloride, 29 mM sucrose and 48 mM sodium chloride at a pH value of about 6.3.

The pharmaceutical composition may also comprise or consist of 0.3 mM Etanercept, 25 mM citrate buffer, e.g. sodium citrate, 25 mM lysine, e.g. lysine hydrochloride, 29 mM sucrose and 88 mM sodium chloride at a pH value of about 6.3.

The pharmaceutical composition may also comprise or consist of 0.3 mM Etanercept, 25 mM citrate buffer, e.g. sodium citrate, 25 mM proline, 29 mM sucrose and 88 mM sodium chloride at a pH value of about 6.3.

The pharmaceutical composition may also comprise or consist of 0.3 mM Etanercept, 25 mM citrate buffer, e.g. sodium citrate, 25 mM proline, 29 mM sucrose and 100 mM sodium chloride at a pH value of about 6.3.

The pharmaceutical composition may also comprise or consist of 0.3 mM Etanercept, 50 mM citrate buffer, e.g. sodium citrate, 50 mM lysine, e.g. lysine hydrochloride, 29 mM sucrose and 50 mM sodium chloride at a pH value of about 6.3.

The pharmaceutical composition may also comprise or consist of 0.3 mM Etanercept, 75 mM citrate buffer, e.g. sodium citrate, 25 mM lysine, e.g. lysine hydrochloride, 29 mM sucrose and 50 mM sodium chloride at a pH value of about 6.3.

The pharmaceutical composition may also comprise or consist of 0.3 mM Etanercept, 65 mM citrate buffer, e.g. sodium citrate, 25 mM lysine, e.g. lysine hydrochloride, 29 mM sucrose and 55 mM sodium chloride at a pH value of about 6.3.

Preferably, the pharmaceutical composition is a stable liquid composition. The term "stable" as used herein means that the TNFR:Fc exhibits one or more of the following features:
(i) exhibiting less than 99% aggregation products (SUM APs) as compared to the same TNFR:Fc formulated in the commonly used phosphate buffered formulation containing 0,3 mM Etanercept in a matrix consisting of 25 mM phosphate buffer, 25 mM arginine and sodium chloride in a molarity greater than 75 mM or in a the amount needed to adjust isotonicity, more preferably less than 98,5% SUM APs, even more preferably less than 98% SUM APs, most preferably less than 97,5% SUM APs, as determined after three months of storage at 40°C by SEC;
(ii) and/or exhibiting less than 99% degradation products (SUM DPs) as compared to the same TNFR:Fc formulated in the commonly used phosphate buffered formulation containing 0,3 mM etanercept in a matrix consisting of 25 mM phosphate buffer, 25 mM Arginine and sodium chloride in a molarity greater than 75 mM or in a the amount needed to adjust isotonicity, more preferably less than 98% SUM DPs, such as less than 97% SUM DPs, even more preferably less than 96% SUM DPs, such as less than 95% SUM DPs, most preferably less than 94% SUM DPs, such as less than 93% SUM DPs, as determined after three months of storage at 40°C by SEC.

Thus, the pharmaceutical formulation according to the invention may be suitably formulated for long term storage. As used herein, the term "long term storage" shall refer to storage of a composition comprising the pharmaceutical formulation for more than 1 month, preferably for more than 2, 3, 4, 5, 6, 7, 8, 9, 10, 11 or even 12 months.

As exemplified below, stability of formulations containing either a citrate or phosphate buffer system in combination with either lysine or proline as stabilizer were assessed during a three month-stability study at intended (2-8 °C) as well as accelerated storage condition (25 and 40°C). Formulations containing the citrate buffer system were thereby determined to be superior compared to formulations containing the phosphate buffer system regarding the formation of post peaks (as determined e.g. by RPC), the formation of degradation products (as determined e.g. by SEC) and the formation of acid peaks (as determined e.g. by CEX).

The described effects were partially even more pronounced, including the superior stabilization of TNFR:Fc formulated in the citrate/lysine formulation matrix, if formulations were stored in vials, displaying a greater liquid/air interaction surface. Determination of the stability of a liquid composition is further exemplified in the examples section, and in particular in Example 3.

The pharmaceutical composition according to the first aspect can be used in a medical treatment. Diseases which may be treated by using the pharmaceutical composition of the invention include, but are not limited to autoimmune diseases, ankylosing spondylitis, juvenile rheumatoid arthritis, psoriasis, psoriatic arthritis, rheumatoid arthritis, Wegener's disease (granulomatosis), Crohn's disease or inflammatory bowel disease, chronic obstructive pulmonary disease (COPD), Hepatitis C, endometriosis, asthma, cachexia, atopic dermatitis, Alzheimers disease and cancer. Accordingly, also contemplated is a method of treatment, comprising administering the composition according to the first aspect to a subject in need thereof, e.g. a subject suffering from one of the above mentioned diseases.

Dosage of the TNFR:Fc will depend on the disease, severity of condition, patient's clinical history, and response to the (prior) therapy, and will be adjusted and monitored by a physician. The pharmaceutical composition may be administered parenterally, such as subcutaneously, intramuscularly, intravenously, intraperitoneally, intracerebrospinally, intra-articularly, intrasynovially and/or intrathecally by either bolus injection or continuous infusion.

The dosage of TNFR:Fc per adult may range from about 1-500 mg/m², or from about 1-200 mg/m², or from about 1-40 mg/m² or about 5-25 mg/m². Alternatively, a flat dose may be administered, wherein the amount may range from 2-500 mg/dose, 2-100 mg/dose or from about 10-80 mg/dose. The dose may be administered more than one time per week, such as two or more times per week at a dose range of 25-100 mg/dose. In another embodiment, an acceptable dose for administration by injection contains 80-100 mg/dose, e.g. 80 mg per dose. The doses can be administered weekly, biweekly or separated by several weeks, e.g. by three weeks.

It is further contemplated that an improvement of the patient's condition will be obtained by a dose of up to 100 mg of the pharmaceutical composition one to three times per week over a period of at least three weeks, though treatment for longer periods may be necessary to induce the desired degree of improvement. However, for incurable chronic conditions the regimen may be continued indefinitely. A suitable regimen for paediatric patients (ages 4-17) may involve a dose of 0.4 mg/kg to 5 mg/kg of TNFR:Fc, administered one or more times per week.

More specifically, in the case of rheumatoid arthritis, 25 mg TNFR:Fc may be administered twice weekly. Alternatively, 50 mg administered once weekly has been shown to be safe and effective.

In the case of psoriatic arthritis and ankylosing spondylitis, the recommended dose is 25 mg TNFR:Fc administered twice weekly or 50 mg administered once weekly. Turning to plaque psoriasis, the recommended dose of TNFR:Fc is 25 mg administered twice weekly or 50 mg administered once weekly. Alternatively, 50 mg given twice weekly may be used for up to 12 weeks followed, if necessary, by a dose of 25 mg twice weekly or 50 mg once weekly. Treatment with TNFR:Fc should continue until remission is achieved, for up to 24 weeks. Continuous therapy beyond 24 weeks may be appropriate for some adult patients. Treatment should be discontinued in patients who show no response after 12 weeks. If re-treatment with TNFR:Fc is indicated, the same guidance on treatment duration should be followed. The dose should be 25 mg twice weekly or 50 mg once weekly.

Regarding juvenile idiopathic arthritis (age 4 years and above), 0.4 mg/kg (up to a maximum of 25 mg per dose) after reconstitution of 25 mg TNFR:Fc in 1 ml of solvent, may be given twice weekly as a subcutaneous injection with an interval of 3-4 days between doses.

Concerning paediatric plaque psoriasis (age 8 years and above), 0.8 mg/kg (up to a maximum of 50 mg per dose) once weekly for up to 24 weeks may be administered. Treatment should be discontinued in patients who show no response after 12 weeks. If re-treatment with TNFR:Fc is indicated, the above guidance on treatment duration should be followed. The dose should be 0.8 mg/kg (up to a maximum of 50 mg per dose) once weekly.

In case of renal and hepatic impairment no dose adjustment is required.

In a second aspect, the invention relates to a kit comprising a composition according to the first aspect and instructions for use of the present composition, as defined in the claims.

In a preferred embodiment, the composition is contained in a pre-filled syringe. In another preferred embodiment, the composition is contained in a pre-filled vial. The kit may comprise one or more unit dosage forms containing the pharmaceutical composition of the invention. Examples for suitable syringes are BD Hypak SCF 1ml long, glass pre-fillable syringes assembled with PTFE coated stoppers (rubber quality 4023/50 from West). The glass vials may be for example 6R glass vials (hydrolytic class I) assembled with PTFE coated stoppers (rubber quality 4023/50 from West). However, any other suitable syringe or vial may be used. The kit may also comprise the pharmaceutical composition according to the invention in another secondary container, such as in an autoinjector.

The prefilled syringe may contain the formulation in aqueous form. Described syringe may be further supplied with an autoinjector, which often is a disposable article for single use only, and may e.g. have a volume between 0.1 and 1 ml. However, the syringe or autoinjector may also be for multi-usage or multi-dosing. The described vial may contain the formulation in lyophilised or aqueous state, and may serve as a single or multiple use device. The vial may e.g. have a volume between 1 and 10 ml. In a third aspect, the invention pertains to a method of producing a pharmaceutical composition according to the first aspect, comprising combining TNFR:Fc, a citrate buffer and an amino acid selected from the group consisting of lysine and proline and their pharmaceutical acceptable salts.

In one particular embodiment, the method may comprise a further step of adding a pharmaceutically acceptable solvent as defined above. The method may further comprise the step of adding at least one tonicity modifier, such as sodium chloride and/or sucrose, and optionally an excipient as defined above. In a final preferred embodiment, the method may further comprise a lyophilization step, which step may be before or after adding the at least one tonicity modifier, and/or an excipient as defined above.

The invention will be more fully understood by reference to the following examples.

### EXAMPLES

### Description of Materials

The TNFR:Fc which was used for the described examples, is derived from recombinant CHO cells, which have been cultured in a fed-batch process in chemically defined medium. The TNFR:Fc is purified from the cell free harvest by standard methods including affinity chromatography on protein A resins and by further chromatographic and filtration steps.

### Description of methods

### General

All chromatographic methods were performed on Agilent 1100 and 1200 HPLC systems equipped with UV and fluorescence detection.

### Size exclusion chromatography (SEC)

Size exclusion chromatography was used to separate lower and higher molecular mass variants of TNFR:Fc as well as any impurities and formulation ingredients. The results were described as the summation of aggregation peaks (APs) and summation of degradation peaks (DPs). In SEC, the identity of test samples was determined by comparing the chromatographic retention time of the major peaks with the retention time of the major peak of a reference standard.

SEC was performed using two sequential Tosoh Bioscience TSK-Gel G3000SWXL columns (5 µm, 250 Å, 7.8 mm I.D. x 300 mm length) (Tosoh Bioscience, Stuttgart, Germany) and a mobile phase containing 150 mM potassium phosphate, pH 6.5. The flow rate was set to 0.4 ml/min and the column temperature to 30°C. Samples were diluted with mobile phase to a concentration of 0.75 mg/ml and injection volume was 10 µl.

### Reverse Phase Chromatography (RP-HPLC or RPC)

The content of samples was determined via RP-HPLC using a C8 column (5 µm, 300 Å, 2.1 mm I.D. x 75 mm length) at 70°C column temperature. Separation of product variants was achieved by applying a linear gradient from 20 to 30% mobile phase B (mobile phase A: 10 % acetonitrile, 0.3 % PEG 300, 0.1 % TFA; mobile phase B: 90 % acetonitrile, 0.3 % PEG 300, 0.1 % TFA) at a flow rate of 1.0 ml/min. Samples were desialylated and diluted to a concentration of 2.5 mg/ml before injection. Chromatograms of UV detection were used for evaluation of purity. Results were displayed as content as well as post peaks (Sum PPs).

### Cation exchange chromatography (CEX)

Cation-exchange separation was performed using a silica based cation exchange resin with bonded coating of polyaspartic acid (100 mm length x 4.6 mm i.d.; 3 µm particles). Before injection, samples were desialylated and concentration was adjusted to 2.5 mg/ml with desialylation buffer. Samples were eluted by a linear gradient from 30 to 50% mobile phase B over 20 min (A: 50 mM sodium acetate, pH 5.2; B: 50 mM sodium acetate, 250 mM NaCl, pH 5.2). The flow rate and temperature were set to 1.0 ml/min and 30°C, respectively. Chromatograms from UV detection were used for data evaluation. The results were displayed as summation of acidic peaks (Sum APs) and summation of basic peaks (Sum BPs)

### Particle counting (PC) method

Particle counting was performed by light obscuration, utilizing an Accusizer Nicomb SIS 780 instrument. This technique detects the light scattered by a particle/aggregate within a liquid environment. The signal will be calculated into the hydrodynamic radius for the detected particle/aggregate. A mean out of three measurements was calculated utilizing a total sample volume of 2.8 ml. The following instrument parameters were used: Range: 0.5 - 500 µm (512 channel; logarithmic scale); flow rate: 5 ml/min.

The influence of the excipients sucrose, arginine and NaCl on the aggregation of TNFR:Fc was evaluated prior to performing the tests described in example 1-3. The resulting formulations were subjected to a stress stability study including stirring and shaking. Aggregates with a radius up to 200nm were detected via SEC method, while particle counting determines particles in the range of 500 nm to 400 µm.

### Arginine:

Analysis of stressed formulations via SEC, revealed the reduction of aggregates due to the addition of arginine. At the same time the formation of bigger sized particles with increasing molarity of arginine was detected via the PC method. It can therefore be concluded that increasing amounts of arginine in TNFR:Fc formulations facilitate the decrease of smaller sized particles (r= up to 200 nm), which is most probably due to the clustering of aggregates to bigger sized particles which are only detectable via the particle counting method.

### NaCl:

The addition of higher NaCl molarities led to the same PC results as for the addition of arginine. The amount of DPs, detected via SEC method, increased with increasing molarities of NaCl

### Sucrose:

The addition of increasing molarities of sucrose, seemed to lead to the reduction of the detected radius of paricles via PC method. No significant results were detected via SEC.

### Example 1

### Formulation Screening

The evaluation was performed by a combination of a DoE (design of experiments) and single parameter studies. All formulations contained 50 mg/ml TNFR:Fc, 100 mM NaCl and were adjusted to a pH of around pH 6.3. The formulations were divided into groups called surfactants, buffer, stabilizer I (containing sugars and sugar alcohols) as well as stabilizer II (amino acids), whereas the stabilizer groups were designed and evaluated by a statistical design (Table 3). The results from these groups were evaluated for their statistical significance. Results were in addition compared to the prior art formulation corresponding to Enbrel® (Table 1) of theTNFR:Fc.

**Table 3. Evaluated factors during formulation screening of TNFR:Fc**

| ***Evaluated factors*** | ***Applied range*** |
|---|---|
| ***Sugars - Stabilizer I*** | |
| Sucrose | 0-100 mM |
| Trehalose | 0-100 mM |
| Sorbit | 0-100 mM |

| ***Amino acids - Stabilizer II*** | |
|---|---|
| Glycine | 0-100 mM |
| Lysine | 0-100 mM |
| Proline | 0-100 mM |
| Arginine | 0 / 100 mM |

| ***Buffer*** | |
|---|---|
| Phosphate | 0 / 25 mM |
| Succinate | 0 / 25 mM |
| Histidine | 0 / 25 mM |
| Citrate | 0/25 mM |

| ***Tonicity agent*** | |
|---|---|
| NaCl | 100 / 150 mM |

The resulting formulations (Table 4) were mechanically stressed and subjected to a short term stress stability study (Table 5). As reference served the Etanercept formulation (i.e. as described in table 1).

**Table 4. Formulation evaluated during formulation screening phase. The set up combines a single parameter evaluation of buffers (formulation 1 - 4) as well as a DoE approach for stabilizer group I including sugars or sugar alcohols (formulation 5 - 9) and stabilizer group II including amino acids (formulation 10 - 14). In addition one formulation similar to prior art formulation corresponding to Enbrel® but omitting arginine (formulation 18) as well as one formulation omitting arginine and adding a higher concentration of NaCl (formulation 19) were tested. The prior art formulation corresponding to Enbrel® served as control (formulation 1).**

| | Buffer | | | | DoE | | | | | DoE | | | | | Single parameter | |
|---|---|---|---|---|---|---|---|---|---|---|---|---|---|---|---|---|
| | | | | | Stabilizer I | | | | | Stabilizer II | | | | | omitting Arginine | omitting Arginine, including NaCl |
| | 1 | 2 | 3 | 4 | 5 | 6 | 7 | 8 | 9 | 10 | 11 | 12 | 13 | 14 | 15 | 16 |
| | [mg/ml] | | | | [mg/ml] | | | | | [mg/ml] | | | | | [mg/ml] | |
| Etanercept (M=150000g/mol) | 50 | 50 | 50 | 50 | 50 | 50 | 50 | 50 | 50 | 50 | 50 | 50 | 50 | 50 | 50 | 50 |
| Sucrose | 10 | 10 | 10 | 10 | - | 17.1 | 34,2 | 34,2 | - | 10 | 10 | 10 | 10 | 10 | 10 | 10 |
| Trehalose | - | - | - | - | - | 18,9 | 37,8 | - | 37,8 | - | - | - | - | - | - | - |
| Sorbitol | - | - | - | - | 18,2 | 9,1 | 18,2 | - | - | - | - | - | - | - | - | - |
| NaCl | 5,8 | 5,8 | 5,8 | 5,8 | 5,8 | 5,8 | 5,8 | 5,8 | 5,8 | 5,8 | 5,8 | 5,8 | 5,8 | 5,8 | 5,8 | 8,8 |
| Arginine | 5,3 | 5,3 | 5,3 | 5,3 | - | - | - | - | - | - | - | - | - | - | - | - |
| Proline | - | - | - | - | - | - | - | - | - | 11,5 | 11,5 | 5,8 | - | - | - | - |
| Lysine | - | - | - | - | - | - | - | - | - | - | 14,6 | 7,3 | - | 14,6 | - | - |
| Glycine | - | - | - | - | - | - | - | - | - | - | 7,5 | 3,8 | 7,5 | - | - | - |
| NaH2PO4 | 2,6 | - | - | - | 2,6 | 2,6 | 2,6 | 2,6 | 2,6 | 2,6 | 2,6 | 2,6 | 2,6 | 2,6 | 2,6 | 2,6 |
| Na2HPO4 | 0,9 | - | - | - | 0,9 | 0,9 | 0,9 | 0,9 | 0,9 | 0,9 | 0,9 | 0,9 | 0,9 | 0,9 | 0,9 | 0,9 |
| DiNatriumSuccinate | - | 6,8 | - | - | - | - | - | - | - | - | - | - | - | - | - | - |
| Histidine | - | - | 3,9 | - | - | - | - | - | - | - | - | - | - | - | - | - |
| Citric acid | - | - | - | 5,3 | - | - | - | - | - | - | - | - | - | - | - | - |
| pH | 6,3 | 6,3 | 6,3 | 6,3 | 6,3 | 6,3 | 6,3 | 6,3 | 6,3 | 6,3 | 6,3 | 6,3 | 6,3 | 6,3 | 6,3 | 6,3 |

**Table 5. Analytical methods and stress conditions applied during formulation screening phase. Rotations per minute (rpm); Agitation per minute (apm)**

| ***Methods*** | ***Stress conditions*** | ***Pull points*** |
|---|---|---|
| ***Shaking (180 apm*)*** | | |
| SEC | Formulation 1 - 20 | 6 hours |
| SEC | Formulation 1 - 20 | 16 hours |

| ***Stirring (600 rpm**)*** | | |
|---|---|---|
| SEC | Formulation 1 - 20 | 6 hours |
| SEC | Formulation 1 - 20 | 16 hours |

| ***Freeze* / *Thaw cycles*** | | |
|---|---|---|
| SEC | Formulation 1, 2, 3 and 20 | 1 cycle |
| SEC | Formulation 1, 2, 3 and 20 | 3 cycles |

| ***Temperatures*** | | |
|---|---|---|
| SEC, CEX, RPC | Formulation 3 - 20 | 0 weeks |
| SEC, CEX, RPC | Formulation 3 - 20 | 2 weeks |
| SEC, CEX, RPC | Formulation 3 - 20 | 4 weeks |

A positive influence on the quality attributes of TNFR:Fc for the out parameters RPC, CEX and SEC was defined as:
- Content (RPC) - no decresae
- Sum APs (CEX) - low level
- Sum BPs (CEX) - comparable to prior art based formulation
- Sum APs (SEC) - low level
- Sum DPs (SEC) - low level

Results were in addition compared to the TNFR:Fc in the prior art formulation corresponding to Enbrel® and marked positive if they were at least comparable to the results of the prior art formulation corresponding to Enbrel® or if they were exceeding them according to the above mentioned parameters. A positive influence on the TNFR:Fc stability in formulation showed the evaluated factors proline, lysine, sucrose and citrate (Table 6).

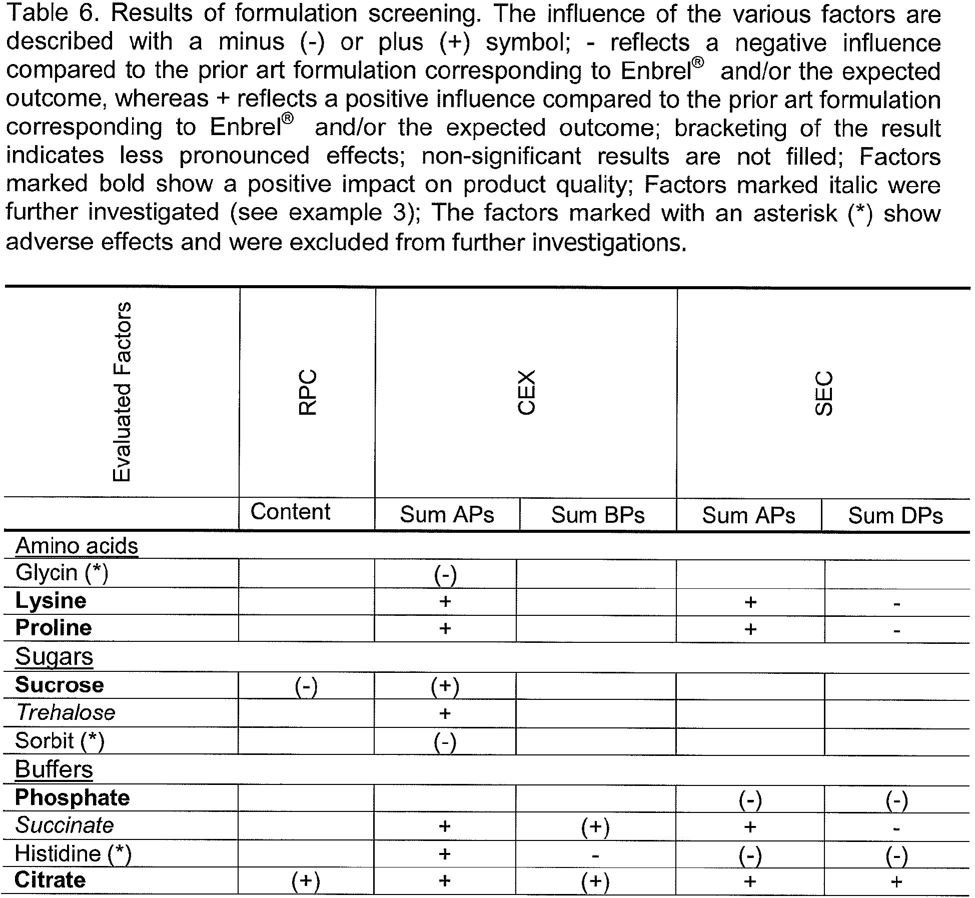

### Example 2

### Focused Screening

Selected factors from the formulation screening that provided significant effects were further evaluated by combining them in another DoE approach (formulation 1-13; factors: proline, lysine, phosphate, citrate) as well as formulations varying single parameters (Lysine/ succinate (formulation 15); Proline/ succinate (formulation 16); Lysine/ citrate/ poloxamer (formulation 17); Trehalose/ citrate/ lysine (formulation 18)). Formulation 19 displays the prior art formulation corresponding to Enbrel® formulation.

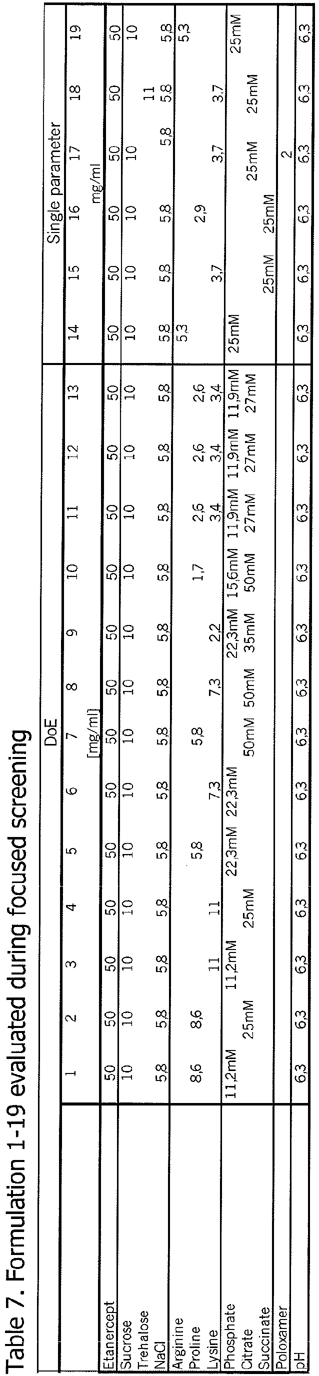

Resulting formulations were subjected to a short term stress stability study (Table 8). The analytical methods SEC, CEX and RPC were applied.

**Table 8. Analytical methods and stress conditions applied during focused screening**

| ***Methods*** | ***Stress conditions*** | ***Pull points*** |
|---|---|---|
| ***Stirring (600 rpm)*** | | |
| SEC | Formulation 1 - 19 | 48 hours |

| ***Freeze* / *Thaw cycles*** | | |
|---|---|---|
| SEC | Formulation 1 - 19 | 3 cycles |

| ***Temperatures*** | | |
|---|---|---|
| SEC | Formulation 1 - 13 | 0 weeks |
| SEC | Formulation 1 - 13 | 2 weeks |
| SEC | Formulation 1 - 13 | 4 weeks |
| SEC, CEX, RPC | Formulation 14 - 19 | 0 weeks |
| SEC, CEX, RPC | Formulation 14 - 19 | 2 weeks |
| SEC, CEX, RPC | Formulation 14 - 19 | 4 weeks |

The factors lysine, proline, citrate buffer and phosphate buffer were evaluated regarding the formation of degradation products due to the storage of TNFR:Fc formulations at 25 (A) and 40°C (B), as well as due to applied stress during stirring for 48 h (C) and three freezing and thawing cycles (D). A positive influence on the quality attributes of TNFR:Fc for the out parameters SEC was defined as:
- Sum DPs (SEC) - low level, no increase

A significantly positive effect was determined for the factor citrate buffer, which led to low degradation product levels in the resulting TNFR:Fc formulations at the in table 8 described conditions. Besides that only proline showed a slightly significant effect at storage at 25°C and after 48h of stirring at 600 rpm, where degradation products were increasing with the applied stress.

The evaluation of the excipients succinate buffer, in combination with lysine or proline, poloxamer and trehalose were evaluated via RPC, SEC and CEX analysis (Table 9). A positive influence on the quality attributes of TNFR:Fc was defined as:
- Content (RPC) -no decrease
- Sum APs (CEX) - low level
- Sum DPs (SEC) - low level

The formulations containing succinate (formulation 15 (including lysine), formulation 16 (including proline)) showed the formation of additional degradation peaks at 40°C storage. Thus, succinate was excluded from further evaluations. As the surfactant poloxamer and the stabilizer trehalose did not show a superior impact on stability (in the range of the prior art formulation corresponding to Enbrel®), poloxamer and trehalose were also excluded form further evaluations.

**Table 9. Summary of influences of excipients succinate, poloxomer and trehalose evaluated during focused screening.**

| | Succinate | | Poloxamer | Trehalose | Target |
|---|---|---|---|---|---|
| | Lysin | Proline | | | |
| Content (RPC) | Decrease | Decrease | No decrease | No decrease | No decrease |
| DPs (SEC) | Increase | Increase | Low level | Low level | Low level |
| APs (CEX) | Increase | Increase | Low level | Low level | Low level |

### Example 3

### Formulation optimization

The most promising four formulations (formulation 1 - 4) from prior evaluations were subjected to a long term stability study at intended (2-8°C) as well as accelerated (25 and 40°C) storage conditions. The long term stability study was performed with TNFR:Fc formulation filled in syringes (0,5 ml) and vials (1 ml). Table 10 gives an overview on the tested formulations. As reference served the prior art formulation corresponding to Enbrel® (formulation 5). All formulations were compared to each other as well as to the described reference.

**Table 10. Overview tested formulations formulation optimization**

| **Batch** | **Strength [mg/ml]** | **Composition** | **Fill volume [ml]** | **Container** |
|---|---|---|---|---|
| 1030ML220_1_s | 50 | 50 mM citrate, 29 mM sucrose, 25 mM NaCl, 25 mM L-Lysine, pH 6.3 | 0.5 | syringe |
| 1030ML220_2_s | 50 | 25 mM citrate, 29 mM sucrose, 63 mM NaCl, 25 mM L-Lysine, pH 6.3 | 0.5 | syringe |
| 1030ML220_3_s | 50 | 25 mM citrate, 29 mM sucrose, 75 mM NaCl, 25 mM L-Prolin, pH 6.3 | 0.5 | syringe |
| 1030ML220_4_s | 50 | 25 mM phosphate, 29 mM sucrose, 75 mM NaCl, 25 mM L-Lysine, pH 6.3 | 0.5 | syringe |
| 1030ML220_5_s | 50 | 25 mM phosphate, 29 mM sucrose, 75 mM NaCl, 25 mM L-pH 6.3 | 0.5 | syringe |
| 1030ML220_1_v | 50 | 50 mM citrate, 29 mM sucrose, 25 mM NaCl, 25 mM L-Lysine, pH 6.3 | 1.0 | 2R vial |
| 1030ML220_2_v | 50 | 25 mM citrate, 29 mM sucrose, 63 mM NaCl, 25 mM L-Lysine, pH 6.3 | 1.0 | 2R vial |
| 1030ML220_3_v | 50 | 25 mM citrate, 29 mM sucrose, 75 mM NaCl, 25 mM L-Prolin, pH 6.3 | 1.0 | 2R vial |
| 1030ML220_4_v | 50 | 25 mM phosphate, 29 mM sucrose, 75 mM NaCl, 25 mM L-Lysine, pH 6.3 | 1.0 | 2R vial |
| 1030ML220_5_v | 50 | 25 mM phosphate, 29 mM sucrose, 75 mM NaCl, 25 mM L-Arginine, pH 6.3 | 1.0 | 2R vial |

A positive effect for the evaluated factors lysine and proline as stabilizer as well as citrate and phosphate buffers systems was declared if the stability for output parameters summation of post peak (RPC), summation of aggregation and degradation products (SEC) as well as summation of acid peaks (CEX) was exceeded compared to the reference. The targets for output parameters are described in Table 11.

**Table 11. Target description during formulation optimization**

| Output parameter | Method | Target |
|---|---|---|
| Summation of post peaks | RPC | Low level |
| Summation of aggregation products | SEC | Low level |
| Summation of degradation products | SEC | Low level |
| Summation of acidic peaks | CEX | Low level |

Results for the output parameters RPC / summation of post peak (PP), SEC / summation of aggregation (APs) and degradation products (DPs) and CEX / summation of acid peaks (APs) are displayed in Table 12, 13 (storage at 2-8°C), Table 14, 15 (storage at 25°C) as well as Table 16, 17 (storage at 40°C). Analysis of samples was performed at start of stability study as well as after 1, 2 and 3 month storage at the declared temperatures.

### Result summary for TNFR:Fc formulation stored at 2-8, 25 and 40°C for three month (Table 12)

### 1) RPC - Summation of post peaks (Sum PPs)

After three month storage at 2-8, 25 and 40°C, TNFR:Fc formulations containing a citrate buffer system (formulation 1-3) displayed lower values of post peaks compared to formulations containing a phosphate buffer (formulation 4 and 5). The absolute lowest value was detected for the formulation containing 50mM citrate buffer and 25 mM lysine (formulation 1). The described effect was even more pronounced for TNFR:Fc formulations stored in vials.

### 2) SEC - Summation of aggregation products (Sum APs)

After 3 month storage at 2-8, 25 and 40°C, TNFR:Fc formulations 1 to 5 are comparable regarding the amount of aggregation products. In addition no difference was detected between formulations filled in vials or syringes.

### 3) SEC - Summation of degradation products (Sum DPs)

After three month storage at 2-8, 25 and 40°C TNFR:Fc formulations containing a citrate buffer system (formulation 1-3) displayed significantly lower values of degradation products compared to formulations containing a phosphate buffer (formulation 4 and 5). The absolute lowest value was detected for the formulation containing 50mM citrate buffer and 25 mM lysine (formulation 1). The described effect was even more pronounced for TNFR:Fc formulations stored in vials.

### 4) CEX - Summation of acidic peaks (Sum APs)

After three month storage at 2-8, 25 °C and 40°C TNFR:Fc formulations in pre-filled syringes, containing a citrate buffer system (formulation 1-3) displayed significantly lower values of acidic peaks compared to formulations containing a phosphate buffer (formulation 4 and 5). The described effect was more pronounced for TNFR:Fc formulations stored at 2-8°C in vials.

### Conclusion formulation optimization

Stability of formulations 1 to 5 during three month storage at 2-8, 25 and 40°C, was assessed via RPC, SEC and CEX analysis. Formulations containing a citrate buffer system (formulation 1- 3) were determined to be superior compared to formulations containing a phosphate buffer system (formulation 4 and 5) regarding the formation of post peaks (RPC), the formation of degradation products (SEC) and the formation of acid peaks (CEX). The absolute best results were determined via RPC and SEC for the formulation containing a 50 mM citrate buffer system and 25 mM lysine as stabilizer. Among the worst, if not the worst formulation was the reference, prior art formulation corresponding to Enbrel® (formulation 5). The degradation of TNFR:Fc, determind via RPC, SEC and CEX was in general more pronounced if formulations were stored in vials. This is most probably due to the greater liquid/air interaction surface displayed by vials compared to syringes. The effects were therefore partially even more pronounced, including the superior stabilization of TNFR:Fc formulated in the citrate / lysine formulation matrix.

**Table 12. Summary results of formulation optimization**

| | RPC Sum PPs | SEC | | CEX Sum APs |
|---|---|---|---|---|
| | | Sum APs | Sum DPs | |
| 2-8°C | Formulations containing citrate display significantly lower values than the ones containing phosphate; Vials: effect more pronounced; Best formulation: 50mM Citrate/25mM Lysine; | Formulation 1 to 5 are comaparable | Formulations containing citrate display significantly lower values than the ones containing phosphate; Vials: effect more pronounced; Best formulation: 50mM Citrate/25mM Lysine; | Formulations containing citrate display significantly lower values than the ones containing phosphate; Vials: effect more pronounced; |
| 25°C | See 2-8°C | See 2-8°C | See 2-8°C | Formulations containing citrate display significantly lower values than the ones containing phosphate; |
| 40°C | See 2-8°C | See 2-8°C | See 2-8°C | See 25°C |

### TNFR:Fc stored at 2-8°C - syringes and vials

**Table 13. Syringes - 2-8°C**

| **RPC Sum PPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 10,2 | 10,2 | 10,2 | 10,3 | 10,3 |
| 3M | 11,2 | 11,6 | 11,7 | 12,5 | 12,7 |
| | | | | | |

| **SEC Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 0,7 | 0,8 | 0,7 | 0,7 | 0,7 |
| 1M | 0,7 | 0,7 | 0,7 | 0,6 | 0,7 |
| 2M | 0,8 | 0,8 | 0,8 | 0,8 | 0,8 |
| 3M | 0,7 | 0,8 | 0,8 | 0,8 | 0,8 |
| | | | | | |

| **SEC Sum DPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 4,0 | 4,1 | 3,9 | 4,4 | 4,4 |
| 1M | 4,8 | 4,8 | 4,9 | 7,4 | 7,5 |
| 2M | 6,0 | 6,2 | 6,1 | 10,3 | 10,0 |
| 3M | 6,0 | 6,5 | 6,5 | 12,8 | 12,8 |

| **CEX Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 9,3 | 8,3 | 9,1 | 8,2 | 9,2 |
| 3M | 10,0 | 10,5 | 10,8 | 12,7 | 13,1 |

**Table 14. Vials - 2-8°C**

| **RPC Sum PPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 10,1 | 10,2 | 10,2 | 10,3 | 10,3 |
| 3M | 11,5 | 11,9 | 12,1 | 13,1 | 13,0 |
| | | | | | |

| **SEC Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 0,7 | 0,7 | 0,7 | 0,6 | 0,7 |
| 1M | 0,6 | 0,6 | 0,6 | 0,5 | 0,6 |
| 2M | 0,7 | 0,7 | 0,7 | 0,7 | 0,7 |
| 3M | 0,8 | 0,8 | 0,8 | 0,7 | 0,7 |
| | | | | | |

| **SEC Sum DPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 4,0 | 4,1 | 4,3 | 4,7 | 4,9 |
| 1M | 6,2 | 6,3 | 6,5 | 8,8 | 9,0 |
| 2M | 6,5 | 7,7 | 8,8 | 12,9 | 13,5 |
| 3M | 6,1 | 9,6 | 9,9 | 17,1 | 17,4 |
| | | | | | |

| **CEX Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 8,2 | 9,0 | 8,3 | 9,1 | 8,8 |
| 3M | 11,2 | 11,5 | 12,5 | 13,4 | 14,3 |

### TNFR:Fc stored at 25°C - syringes and vials

**Table 15. Syringes - 25°C**

| **RPC Sum PPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 10,2 | 10,2 | 10,2 | 10,3 | 10,3 |
| 1M | 11,1 | 11,4 | 11,4 | 12,2 | 12,4 |
| 2M | 11,9 | 12,3 | 12,3 | 14,0 | 14,0 |
| 3M | 13,3 | 13,9 | 13,7 | 16,4 | 16,1 |
| | | | | | |

| **SEC Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 0,0 | 0,0 | 0,0 | 0,0 | 0,0 |
| 1M | 1,0 | 1,0 | 1,0 | 1,0 | 1,0 |
| 2M | 1,7 | 1,7 | 1,7 | 1,7 | 1,7 |
| 3M | 1,9 | 2,0 | 2,0 | 2,0 | 1,9 |

| **SEC Sum DPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 0,0 | 0,0 | 0,0 | 0,0 | 0,0 |
| 1M | 8,3 | 8,7 | 8,3 | 13,4 | 12,8 |
| 2M | 9,8 | 10,8 | 10,7 | 18,1 | 17,6 |
| 3M | 11,6 | 12,8 | 12,6 | 21,3 | 20,7 |
| | | | | | |

| **CEX Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 0,0 | 0,0 | 0,0 | 0,0 | 0,0 |
| 1M | 10,0 | 10,7 | 10,4 | 12,6 | 12,8 |
| 2M | 12,7 | 13,1 | 12,9 | 15,2 | 15,3 |
| 3M | 12,9 | 13,4 | 13,5 | 17,8 | 18,5 |

**Table 16. Vials - 25°C**

| **RPC Sum PPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 10,1 | 10,2 | 10,2 | 10,3 | 10,3 |
| 1M | 11,4 | 11,9 | 11,8 | 11,7 | 14,1 |
| 2M | 12,0 | 12,8 | 12,9 | 17,4 | 17,8 |
| 3M | 13,3 | 14,1 | 14,3 | 19,2 | 20,7 |
| | | | | | |

| **SEC Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 0,0 | 0,0 | 0,0 | 0,0 | 0,0 |
| 1M | 0,9 | 1,0 | 0,9 | 0,9 | 0,8 |
| 2M | 1,6 | 1,6 | 1,6 | 1,4 | 1,3 |
| 3M | 1,9 | 2,0 | 2,0 | 1,6 | 1,5 |
| | | | | | |

| **SEC Sum DPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 0,0 | 0,0 | 0,0 | 0,0 | 0,0 |
| 1M | 8,9 | 10,1 | 10,0 | 23,5 | 24,3 |
| 2M | 11,2 | 12,6 | 12,4 | 30,9 | 32,5 |
| 3M | 13,3 | 14,8 | 14,4 | 36,5 | 39,0 |
| | | | | | |

| **CEX Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 0,0 | 0,0 | 0,0 | 0,0 | 0,0 |
| 1M | 10,5 | 10,5 | 10,9 | 10,7 | 15,8 |
| 2M | 12,8 | 13,7 | 13,7 | 19,6 | 19,9 |
| 3M | 14,3 | 15,4 | 12,0 | 14,2 | 14,2 |

### TNFR:Fc stored at 40°C - syringes and vials

**Table 17. Syringes - 40°C**

| **RPC Sum PPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 10,2 | 10,2 | 10,2 | 10,3 | 10,3 |
| 1M | 12,8 | 13,2 | 13,2 | 14,7 | 14,8 |
| 2M | 16,8 | 16,9 | 17,0 | 18,8 | 18,8 |
| 3M | 19,6 | 19,6 | 19,5 | 22,5 | 22,9 |
| | | | | | |

| **SEC Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 0,7 | 0,8 | 0,7 | 0,7 | 0,7 |
| 1M | 3,0 | 3,0 | 3,0 | 3,2 | 3,2 |
| 2M | 3,1 | 3,6 | 3,6 | 3,8 | 3,8 |
| 3M | 7,9 | 8,2 | 7,9 | 8,2 | 8,3 |
| | | | | | |

| **SEC Sum DPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 4,0 | 4,1 | 3,9 | 4,4 | 4,4 |
| 1M | 9,8 | 10,0 | 9,9 | 13,3 | 13,0 |
| 2M | 14,9 | 14,7 | 14,4 | 19,0 | 19,5 |
| 3M | 18,4 | 18,9 | 18,8 | 24,3 | 24,2 |
| | | | | | |

| **CEX Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 9,3 | 8,3 | 9,1 | 8,2 | 9,2 |
| 1M | 13,6 | 13,1 | 13,2 | 15,1 | 14,5 |
| 2M | 18,0 | 19,2 | 18,9 | 20,5 | 19,9 |
| 3M | 28,1 | 27,1 | 26,3 | 29,8 | 30,6 |

**Table 18. Vials - 40°C**

| **RPC Sum PPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 10,1 | 10,2 | 10,2 | 10,3 | 10,3 |
| 1M | 13,3 | 13,0 | 13,0 | 14,9 | 15,2 |
| 2M | 15,8 | 16,2 | 15,6 | 18,7 | 18,5 |
| 3M | 19,1 | 20,0 | 19,6 | 23,9 | 24,1 |
| | | | | | |

| **SEC Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 0,7 | 0,7 | 0,7 | 0,6 | 0,7 |
| 1M | 2,9 | 2,8 | 2,8 | 2,9 | 2,8 |
| 2M | 3,5 | 3,7 | 3,6 | 3,7 | 3,7 |
| 3M | 7,5 | 7,8 | 7,5 | 7,5 | 7,6 |

| **SEC Sum DPs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 4,0 | 4,1 | 4,3 | 4,7 | 4,9 |
| 1M | 10,9 | 11,4 | 11,1 | 15,5 | 15,4 |
| 2M | 15,1 | 15,9 | 15,1 | 21,4 | 20,9 |
| 3M | 19,5 | 20,3 | 19,9 | 27,5 | 27,6 |
| | | | | | |

| **CEX Sum APs** | 50mMCitrate/25mM Lysine | 25 mM Citrate/25 mM Lysine | Citrate 25mM/Proline 25mM | Phosphate 25mM/Lysin 25mM | Phosphate 25mM/Arginine 25mM |
|---|---|---|---|---|---|
| T0 | 8,2 | 9,0 | 8,3 | 9,1 | 8,8 |
| 1M | 14,1 | 14,6 | 13,6 | 15,6 | 15,7 |
| 2M | 19,0 | 19,3 | 18,5 | 20,5 | 21,7 |
| 3M | 28,0 | 26,8 | 26,9 | 29,9 | 32,4 |

## Claims

1. Pharmaceutical composition, comprising 0.15 mM to 0.5 mM TNFR:Fc, a citrate buffer at a concentration from 25 mM to 120 mM at a pH from 6.2 to 6.4, and an amino acid at a concentration from 15 mM to 100 mM selected from the group consisting of lysine and proline and their pharmaceutical acceptable salts; wherein TNFR:Fc is etanercept.

2. The pharmaceutical composition of claim 1, wherein the amino acid is lysine or its pharmaceutical acceptable salt.

3. The pharmaceutical composition of any of the preceding claims, further comprising a tonicity modifier selected from the group consisting of sodium chloride, cysteine, histidine, glycine, potassium chloride, sucrose, glucose and mannitol.

4. The pharmaceutical composition of claim 3, wherein the tonicity modifier is sodium chloride and/or sucrose.

5. The pharmaceutical composition of claim 3 or 4, comprising at least one tonicity modifier at a total concentration from 5 mM to 200 mM.

6. The pharmaceutical composition of any of the preceding claims, further comprising at least one excipient selected from the group consisting of lactose, glycerol, xylitol, sorbitol, mannitol, maltose, inositol, trehalose, glucose, bovine serum albumin (BSA), dextran, polyvinyl acetate (PVA), hydroxypropyl methylcellulose (HPMC), polyethyleneimine (PEI), gelatine, polyvinylpyrrolidone (PVP), hydroxyethylcellulose (HEC), polyethylene glycol (PEG), ethylene glycol, glycerol, dimethysulfoxide (DMSO), dimethylformamide (DMF), L- serine, sodium glutamate, alanine, glycine, sarcosine, gamma-aminobutyric acid (GABA); polyoxyethylene sorbitan monolaurate, preferably Tween-20; polyoxyethylene sorbitan monooleate, preferably Tween-80; sodium dodecyl sulphate (SDS), polysorbate, polyoxyethylene copolymer, potassium phosphate, sodium acetate, ammonium sulfate, magnesium sulfate, sodium sulfate, trimethylamine N-oxide, betaine, zinc ions, copper ions, calcium ions, manganese ions, magnesium ions, CHAPS, sucrose monolaurate, and 2-O-beta-mannoglycerate.

7. The pharmaceutical composition of claim 6, comprising the at least one excipient at a total concentration from 0.1 mM to 0.7 mM.

8. The pharmaceutical composition of any the preceding claims, comprising TNFR:Fc at a concentration of 50 mg/ml.

9. The pharmaceutical composition of any of the preceding claims, wherein said composition further comprises a pharmaceutically acceptable solvent.

10. The pharmaceutical composition of any of the claims 1-8, wherein the composition is lyophilized.

11. The pharmaceutical composition of claim 1,
comprising 15 mM to 100 mM lysine, 10 to 100 mM sucrose and 5 mM to 200 mM sodium chloride at a pH value of about 6.3; or
comprising 15 mM to 100 mM proline, 10 mM to 100 mM sucrose and 5 mM to 200 mM sodium chloride at a pH value of about 6.3.

12. The pharmaceutical composition of claim 1,
comprising 0.3 mM TNFR:Fc, 50 mM citrate buffer, 25 mM lysine, 29 mM sucrose and 75 mM sodium chloride at a pH value of about 6.3; or
comprising 0.3 mM TNFR:Fc, 25 mM citrate buffer, 25 mM lysine, 29 mM sucrose and 88 mM sodium chloride at a pH value of about 6.3; or
comprising 0.3 mM TNFR:Fc, 25 mM citrate buffer, 25 mM proline, 29 mM sucrose and 75 mM sodium chloride at a pH value of about 6.3.

13. Kit comprising a composition of any of the preceding claims and instructions for use of said composition, preferably wherein the composition is contained in a pre-filled syringe or wherein the composition is contained in a pre-filled vial, more preferably wherein the composition is lyophilized and contained in a vial.

14. Method of producing a pharmaceutical composition of any of claims 1 to 12, comprising combining TNFR:Fc, a citrate buffer and an amino acid selected from the group consisting of lysine and proline and their pharmaceutical acceptable salts.

15. The method of claim 14, further comprising adding at least one tonicity modifier, and/or a pharmaceutically acceptable solvent, and optionally an excipient as defined in any one of claims 4 to 7.

16. The method of claim 14 or 15, further comprising a lyophilization step.

17. Composition of any of claims 1 to 12 for use in a medical treatment, in particular in a treatment of diseases selected from the group of diseases consisting of autoimmune disease, ankylosing spondylitis, juvenile rheumatoid arthritis, psoriasis, psoriatic arthritis, rheumatoid arthritis, Wegener's disease (granulomatosis), Crohn's disease or inflammatory bowel disease, chronic obstructive pulmonary disease (COPD), Hepatitis C, endometriosis, asthma, cachexia, atopic dermatitis, Alzheimer and cancer.

## Patentansprüche

1. Pharmazeutische Zusammensetzung, umfassend 0,15 mM bis 0,5 mM TNFR:Fc, einen Zitratpuffer in einer Konzentration von 25 mM bis 120 mM bei einem pH von 6,2 bis 6,4, und eine Aminosäure in einer Konzentration von 15 mM bis 100 mM ausgewählt aus der Gruppe bestehend aus Lysin und Prolin und ihren pharmazeutisch verträglichen Salzen; wobei TNFR:Fc Etanercept ist.

2. Pharmazeutische Zusammensetzung nach Anspruch 1, wobei die Aminosäure Lysin oder sein pharmazeutisch verträgliches Salz ist.

3. Pharmazeutische Zusammensetzung nach einem beliebigen der voranstehenden Ansprüche, die des Weiteren einen Tonizitätsmodifikator umfasst, der aus der Gruppe bestehend aus Natriumchlorid, Cystein, Histidin, Glycin, Kaliumchlorid, Saccharose, Glucose und Mannitol ausgewählt ist.

4. Pharmazeutische Zusammensetzung nach Anspruch 3, wobei der Tonizitätsmodifikator Natriumchlorid und/oder Saccharose ist.

5. Pharmazeutische Zusammensetzung nach Anspruch 3 oder 4, die mindestens einen Tonizitätsmodifikator in einer Gesamtkonzentration von 5 mM bis 200 mM umfasst.

6. Pharmazeutische Zusammensetzung nach einem beliebigen der voranstehenden Ansprüche, die des Weiteren mindestens einen Hilfsstoff umfasst, der aus der Gruppe bestehend aus Lactose, Glycerin, Xylitol, Sorbitol, Mannitol, Maltose, Inositol, Trehalose, Glucose, Rinderserumalbumin (BSA), Dextran, Polyvinylacetat (PVA), Hydroxypropylmethylcellulose (HPMC), Polyethylenimin (PEI), Gelatine, Polyvinylpyrrolidon (PVP), Hydroxyethylcellulose (HEC), Polyethylenglycol (PEG), Ethylenglycol, Glycerin, Dimethylsulfoxid (DMSO), Dimethylformamid (DMF), L-Serin, Natriumglutamat, Alanin, Glycin, Sarkosin, gamma-Aminobuttersäure (GABA); Polyoxyethylensorbitanmonolaurat, bevorzugt Tween-20; Polyoxyethylensorbitanmonooleat, bevorzugt Tween-80; Natriumdodecylsulfat (SDS), Polysorbat, Polyoxyethylen-Copolymer, Kaliumphosphat, Natriumacetat, Ammoniumsulfat, Magnesiumsulfat, Natriumsulfat, Trimethylamin-N-oxid, Betain, Zinkionen, Kupferionen, Calciumionen, Manganionen, Magnesiumionen, CHAPS, Saccharose-Monolaurat, und 2-O-beta-Mannoglycerat ausgewählt ist.

7. Pharmazeutische Zusammensetzung nach Anspruch 6, die den mindestens einen Hilfsstoff in einer Gesamtkonzentration von 0,1 mM bis 0,7 mM umfasst.

8. Pharmazeutische Zusammensetzung nach einem beliebigen der voranstehenden Ansprüche, die TNFR:Fc in einer Konzentration von 50 mg/ml umfasst.

9. Pharmazeutische Zusammensetzung nach einem beliebigen der voranstehenden Ansprüche, wobei die Zusammensetzung des Weiteren ein pharmazeutisch verträgliches Lösungsmittel umfasst.

10. Pharmazeutische Zusammensetzung nach einem beliebigen der Ansprüche 1-8, wobei die Zusammensetzung lyophilisiert ist.

11. Pharmazeutische Zusammensetzung nach Anspruch 1,
umfassend 15 mM bis 100 mM Lysin, 10 bis 100 mM Saccharose und 5 mM bis 200 mM Natriumchlorid bei einem pH-Wert von etwa 6,3; oder
umfassend 15 mM bis 100 mM Prolin, 10 mM bis 100 mM Saccharose and 5 mM bis 200 mM Natriumchlorid bei einem pH-Wert von etwa 6,3.

12. Pharmazeutische Zusammensetzung nach Anspruch 1,
umfassend 0,3 mM TNFR:Fc, 50 mM Zitratpuffer, 25 mM Lysin, 29 mM Saccharose und 75 mM Natriumchlorid bei einem pH-Wert von etwa 6,3; oder
umfassend 0,3 mM TNFR:Fc, 25 mM Zitratpuffer, 25 mM Lysin, 29 mM Saccharose und 88 mM Natriumchlorid bei einem pH-Wert von etwa 6,3; oder
umfassend 0,3 mM TNFR:Fc, 25 mM Zitratpuffer, 25 mM Prolin, 29 mM Saccharose und 75 mM Natriumchlorid bei einem pH-Wert von etwa 6,3.

13. Kit, umfassend eine pharmazeutische Zusammensetzung gemäß einem beliebigen der voranstehenden Ansprüche und Anweisungen zur Verwendung der Zusammensetzung, bevorzugt wobei die Zusammensetzung in einer vorgefüllten Spritze enthalten ist, oder wobei die Zusammensetzung in einem vorgefüllten Fläschchen enthalten ist, stärker bevorzugt wobei die Zusammensetzung lyophilisiert ist und in einem Fläschchen enthalten ist.

14. Verfahren zum Herstellen einer pharmazeutischen Zusammensetzung gemäß einem beliebigen der Ansprüche 1 bis 12, umfassend Kombinieren von TNFR:Fc, einem Zitratpuffer und einer Aminosäure ausgewählt aus der Gruppe bestehend aus Lysin und Prolin und ihren pharmazeutisch verträglichen Salzen.

15. Verfahren nach Anspruch 14, das des Weiteren Zugeben von mindestens einem Tonizitätsmodifikator, und/oder einem pharmazeutisch verträglichen Lösungsmittel, und gegebenenfalls einem Hilfsstoff wie in einem beliebigen der Ansprüche 4 bis 7 definiert umfasst.

16. Verfahren nach Anspruch 14 oder 15, das des Weiteren einen Lyophilisierungsschritt umfasst.

17. Zusammensetzung gemäß einem beliebigen der Ansprüche 1 bis 12 zur Verwendung in einer medizinischen Behandlung, insbesondere in einer Behandlung von Krankheiten, die aus der Gruppe von Krankheiten bestehend aus Autoimmunkrankheit, Spondylitis ankylosans, juveniler rheumatoider Arthritis, Psoriasis, Psoriasis-Arthritis, rheumatoider Arthritis, Wegener-Granulomatose, Morbus Crohn oder chronischentzündlicher Darmerkrankung, chronisch-obstruktiver Lungenerkrankung (COPD), Hepatitis C, Endometriose, Asthma, Kachexie, atopischer Dermatitis, Alzheimer und Krebs ausgewählt sind.

## Revendications

1. Composition pharmaceutique, comprenant 0,15 mM à 0,5 mM de TNFR:Fc, un tampon citrate à une concentration de 25 mM à 120 mM à un pH de 6,2 à 6,4, et un acide aminé à une concentration de 15 mM à 100 mM choisi dans le groupe constitué par la lysine et la proline et leurs sels pharmaceutiquement acceptables ; dans laquelle le TNFR:Fc est l'étanercept.

2. Composition pharmaceutique selon la revendication 1, dans laquelle l'acide aminé est la lysine ou son sel pharmaceutique acceptable.

3. Composition pharmaceutique selon l'une quelconque des revendications précédentes, comprenant en outre un modificateur de la tonicité choisi dans le groupe constitué par le chlorure de sodium, la cystéine, l'histidine, la glycine, le chlorure de potassium, le saccharose, le glucose et le mannitol.

4. Composition pharmaceutique selon la revendication 3, dans laquelle le modificateur de la tonicité est le chlorure de sodium et/ou le saccharose.

5. Composition pharmaceutique selon la revendication 3 ou 4, comprenant au moins un modificateur de la tonicité à une concentration totale de 5 mM à 200 mM.

6. Composition pharmaceutique selon l'une quelconque des revendications précédentes, comprenant en outre au moins un excipient choisi dans le groupe constitué par le lactose, le glycérol, le xylitol, le sorbitol, le mannitol, le maltose, l'inositol, le tréhalose, le glucose, la sérumalbumine bovine (BSA), le dextran, le poly(acétate de vinyle) (PVA), l'hydroxypropylméthylcellulose (HPMC), la polyéthylèneimine (PEI), la gélatine, la polyvinylpyrrolidone (PVP), l'hydroxyéthylcellulose (HEC), le polyéthylène glycol (PEG), l'éthylène glycol, le glycérol, le diméthysulfoxyde (DMSO), le diméthylformamide (DMF), la L-sérine, le glutamate de sodium, l'alanine, la glycine, la sarcosine, l'acide gamma-aminobutyrique (GABA) ; le monolaurate de sorbitan polyoxyéthyléné, de préférence le Tween-20 ; le monooléate de sorbitan polyoxyéthyléné, de préférence le Tween-80 ; le dodécyl sulfate de sodium (SDS), le polysorbate, un copolymère de polyoxyéthylène, le phosphate de potassium, l'acétate de sodium, le sulfate d'ammonium, le sulfate de magnésium, le sulfate de sodium, le N-oxyde de triméthylamine, la bétaïne, les ions zinc, les ions cuivre, les ions calcium, les ions manganèse, les ions magnésium, le CHAPS, le monolaurate de saccharose et le 2-O-bêta-mannoglycérate.

7. Composition pharmaceutique selon la revendication 6, comprenant le au moins un excipient à une concentration totale de 0,1 mM à 0,7 mM.

8. Composition pharmaceutique selon l'une quelconque des revendications précédentes, comprenant du TNFR:Fc à une concentration de 50 mg/ml.

9. Composition pharmaceutique selon l'une quelconque des revendications précédentes, dans laquelle ladite composition comprend en outre un solvant pharmaceutiquement acceptable.

10. Composition pharmaceutique selon l'une quelconque des revendications 1 à 8, dans laquelle la composition est lyophilisée.

11. Composition pharmaceutique selon la revendication 1,
comprenant 15 mM à 100 mM de lysine, 10 à 100 mM de saccharose et 5 mM à 200 mM de chlorure de sodium à une valeur de pH d'environ 6,3 ; ou
comprenant 15 mM à 100 mM de proline, 10 mM à 100 mM de saccharose et 5 mM à 200 mM de chlorure de sodium à une valeur de pH d'environ 6,3.

12. Composition pharmaceutique selon la revendication 1,
comprenant 0,3 mM de TNFR:Fc, 50 mM de tampon citrate, 25 mM de lysine, 29 mM de saccharose et 75 mM de chlorure de sodium à une valeur de pH d'environ 6,3 ; ou
comprenant 0,3 mM de TNFR:Fc, 25 mM de tampon citrate, 25 mM de lysine, 29 mM de saccharose et 88 mM de chlorure de sodium à une valeur de pH d'environ 6,3 ; ou
comprenant 0,3 mM de TNFR:Fc, 25 mM de tampon citrate, 25 mM de proline, 29 mM de saccharose et 75 mM de chlorure de sodium à une valeur de pH d'environ 6,3.

13. Kit comprenant une composition selon l'une quelconque des revendications précédentes et des instructions pour l'utilisation de ladite composition, de préférence dans lequel la composition est contenue dans une seringue pré-remplie ou dans lequel la composition est contenue dans un flacon pré-rempli, de manière davantage préférée dans lequel la composition est lyophilisée et contenue dans un flacon.

14. Procédé de production d'une composition pharmaceutique selon l'une quelconque des revendications 1 à 12, comprenant la combinaison de TNFR:Fc, d'un tampon citrate et d'un acide aminé choisi dans le groupe constitué par la lysine et la proline et leurs sels pharmaceutiques acceptables.

15. Procédé selon la revendication 14, comprenant en outre l'ajout d'au moins un modificateur de la tonicité, et/ou d'un solvant pharmaceutiquement acceptable, et éventuellement d'un excipient tel que défini dans l'une quelconque des revendications 4 à 7.

16. Procédé selon la revendication 14 ou 15, comprenant en outre une étape de lyophilisation.

17. Composition selon l'une quelconque des revendications 1 à 12, pour une utilisation dans un traitement médical, en particulier dans un traitement de maladies choisies dans le groupe de maladies constitué par une maladie auto-immune, la spondylarthrite ankylosante, la polyarthrite rhumatoïde juvénile, le psoriasis, le rhumatisme psoriasique, le rhumatisme articulaire aigu, la maladie de Wegener (granulomatose), la maladie de Crohn ou la maladie intestinale inflammatoire, la bronchopneumopathie obstructive chronique (BPOC), l'hépatite C, l'endométriose, l'asthme, la cachexie, la dermatite atopique, l'Alzheimer et le cancer.
